Europäisches Patentamt

European Patent Office

Office européen des brevets

(11) Numéro de publication: **0 252 837 B1**

## FASCICULE DE BREVET EUROPEEN

(45) Date de publication de fascicule du brevet: **11.11.92** (51) Int. Cl.[5]: **C07H 9/02, A23L 1/00**

(21) Numéro de dépôt: **87401584.5**

(22) Date de dépôt: **07.07.87**

(54) **Procédé de préparation de dianhydrides du fructofuranose et leur utilisation comme additifs alimentaires.**

(30) Priorité: **11.07.86 FR 8610247**

(43) Date de publication de la demande:
**13.01.88 Bulletin 88/02**

(45) Mention de la délivrance du brevet:
**11.11.92 Bulletin 92/46**

(84) Etats contractants désignés:
**AT BE CH DE ES FR GB GR IT LI LU NL SE**

(56) Documents cités:
**WO-A-85/00814**

**CARBOHYDRATE RESEARCH, vol. 136, 1985, pages 53-65, Elsevier Science Publishers B.V., Amsterdam, NL; J. DEFAYE et al.: "The behaviour of d-fructose and inulin towards anhydrous hydrogen fluoride"**

(73) Titulaire: **ERIDANIA BEGHIN-SAY**

**F-59239 Thumeries(FR)**

(72) Inventeur: **Defaye, Jacques**
**202 rue de Vercors**
**F-38330 Saint Ismier(FR)**
Inventeur: **Gadelle, Gerard**
**23 Hameau Fleuri Montbonnot Saint Martin**
**F-38330 Saint Ismier(FR)**
Inventeur: **Pedersen, Christian**
**Bredesvinget 18**
**DK-2830 Virum(DK)**
Inventeur: **Lafont, Didier**
**251 parc de Cassan**
**F-95290 L'Isle Adam(FR)**

(74) Mandataire: **David, Daniel et al**
**KAYSERBERG Service Propriété Industrielle**
**23 boulevard Georges Clemenceau**
**F-92402 Courbevoie Cédex(FR)**

Rank Xerox (UK) Business Services

**Description**

L'invention a pour objet un procédé de préparation de dianhydrides 1,2':2,1'-difructofuranosidiques d'intérêt comme agents édulcorants hypocaloriques et hypocariogènes.

L'article de Defaye, Gadelle et Pedersen, paru dans Carbohydr. Res., 136 (1985) 53-65, rapporte que l'action du fluorure d'hydrogène sur le D-fructose ou l'inuline conduit, en rendement quantitatif, à un mélange d'au moins six dianhydrides dispirodioxanniques qui ont été isolés et caractérisés. WO-A 8 500 814 revendique des méthodes de préparation de ces dianhydrides du fructose, étendues aux céto-hexoses, céto-pentoses, oligo- et poly- saccharides contenant ce motif monosaccharidique, ainsi que leur utilisation comme additifs alimentaires.

L'un des composants du mélange de dianhydrides obtenus par action du fluorure d'hydrogène sur le D-fructose ou l'inuline est l'α-D-fructofuranose β-D-fructofuranose 1,2':2,1'-dianhydride. Il est apparu que ce composé présente un goût sucré agréable. En outre, comme les autres dianhydrides du D- fructose, il n'est pas hydrolysé par la sucrase-isomaltase ; il est donc indiqué tout particulièrement pour une utilisation comme agent édulcorant non calorique.

Le rendement d'obtention de ce composé ne dépasse cependant pas 18 % dans le meilleur des cas, que l'on parte du D-fructose ou d'inuline, et sa séparation des autres isomères présents est difficile car elle fait intervenir en particulier des procédés chromatographiques.

Un dianhydride du D-fructofuranose lié 1,2':2,1' a par ailleurs été obtenu précédemment par action de l'acide nitrique sur le peracétate d'inuline (C. Irvine et J.W. Stevenson, J. Am. Chem. Soc., 51 (1929) 2197-2203 ; L.A. Boggs et F. Smith, J. Am. Chem. Soc., 78 (1956) 1878-1880). Son rendement ne dépasse pas 50 % dans le meilleur des cas. Une structure non symétrique impliquant une anomérie α,β a été ultérieurement proposée sur la base de résultats de r.m.n. ¹H (R.U. Lemieux et R. Nagarajan, Can. J. Chem., 42 (1964) 1270-1278).

Ce composé a également été obtenu avec un rendement faible (environ 5%) par action d'un extrait enzymatique d'Aspergillus fumigatus sur l'inuline (K. Tanaka, K. Sonobe, T, Uchiyama et T. Matsuyama, Carbohydr. Res. 75 (1979) 340-344).

Le but de cette invention est de proposer une méthode commode d'obtention, en bons rendements, de dianhydrides spirodioxanniques dérivés de la forme furanose du D-fructose. L'invention vise en particulier l'α-D-fructofuranose β-D-fructofuranose 1,2':2,1'-dianhydride précédemment mentionné.

Le procédé est caractérisé en ce que l'on fait réagir un glucide comportant au minimum un résidu fructofuranose, substitué sur les positions hydroxyles en C-3, C-4 et C-6 par des fonctions stables en présence du fluorure d'hydrogène, avec le fluorure d'hydrogène utilisé comme solvant du glucide, et réactif, cette étape étant suivie d'une déprotection des positions hydroxyles protégées.

Comme glucide comportant au minimum un résidu fructofuranose, on emploie de préférence l'inuline ou le saccharose. Parmi les substituants des groupes hydroxyles, il est nécessaire de choisir une fonction stable dans le fluorure d'hydrogène ; il pourra s'agir d'une fonction ester, acétate en particulier.

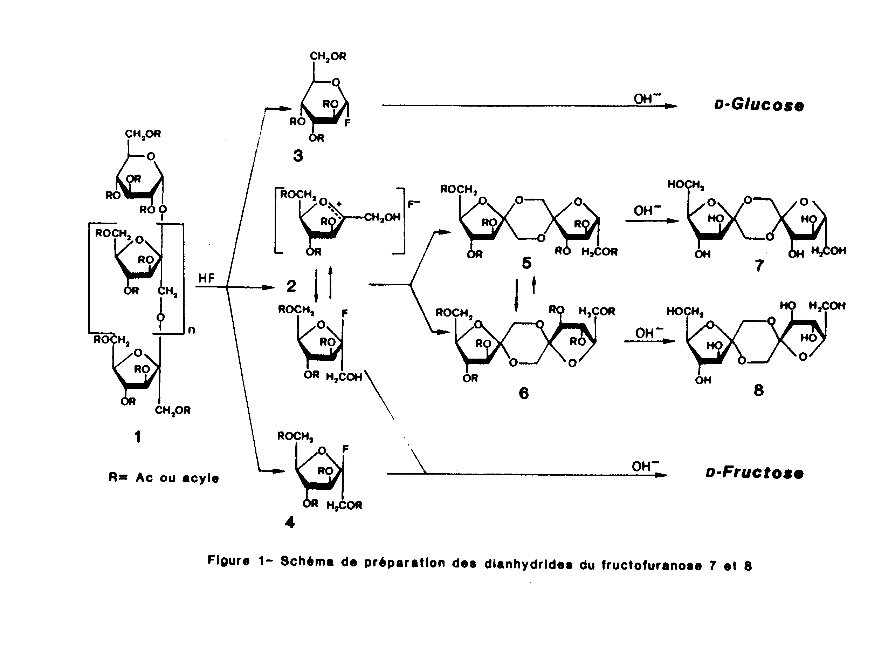

Le mécanisme de la réaction produite par la mise en oeuvre de l'invention est représenté à la figure 1 : le procédé est dans ce cas appliqué à l'inuline substitué par la fonction acétate.

Le procédé de l'invention consiste donc à soumettre le peracétate d'inuline (1) à l'action du fluorure d'hydrogène utilisé comme solvant et réactif. Comme cela est décrit dans l'article de Defaye, Gadelle et Pedersen (loc. cit.), il y a dans une première étape fluorolyse de la liaison interosidique β-(1 → 2) des unités fructofuranosidiques de l'inuline et formation de fluorure de 3,4,6-tri- O-acetyl-β-D-fructofuranosyle (2) en équilibre vraisemblablement avec l'ion fructosyloxocarbénium correspondant. Ce composé se dimérise, en seconde étape, avec formation de 3,4,6:3',4',6'-hexa-O-acetyl di-β-D-fructofuranose dianhydride (5) et de son isomère α,β (6), lequel peut être isolé après neutralisation directe du mélange réactionnel dans l'éther diéthylique. L'hydrolyse des substituants acétate est réalisée par action de quantités catalytiques de méthylate de sodium dans le méthanol ou, de façon plus générale, par action d'agents alcalins tels l'ammoniaque ou la soude. L'α-D-fructofuranose β-D-fructofuranose 1,2':2,1'-dianhydride (8) est obtenu, sous forme cristalline, par cristallisation dans l'éthanol avec des rendements qui, de façon optimale, dépassent 60 %.

Il est également possible d'obtenir l'α-D-fructofuranose β-D-fructofuranose, 1,2':2,1'-dianhydride (8), en mélange avec son isomère β,β (7) si l'on désacétyle directement le produit de la réaction résultant de l'action du fluorure d'hydrogène sur le peracétate d'inuline. Dans ce cas, ce mélange des dianhydrides du fructofuranose (7) et (8) contient également un peu de D-glucose et du D-fructose résultant de réactions impliquant les intermédiaires (3) et (4) telles que décrit dans le schéma de la figure 1, ainsi que de réactions éventuellement incomplètes conduisant à (5) et (6). S'il reste, après désacétylation, des sels minéraux, ils seront aisément éliminés par passage d'une solution aqueuse du mélange sur une colonne échangeuse mixte de cations-anions.

Comme la réaction du fluorure d'hydrogène sur le peracétate d'inuline peut être assez violente et conduire à la formation de produits secondaires non souhaités, ce qui a en particulier pour effet de diminuer le rendement de la réaction en les dianhydrides du fructofuranose (5) et (6), il a été trouvé préférable de diluer ce réactif dans le dioxyde de soufre, ou encore le dioxanne, le premier solvant étant cependant préféré car inerte vis à vis du fluorure d'hydrogène. De même, on opérera à basse température (-20°C) de façon à mieux contrôler la réaction.

Le temps de contact entre le peracétate d'inuline et le fluorure d'hydrogène est un paramètre qui permet de contrôler la proportion relative des deux composants hexa-acétates des $\beta,\beta$- et $\alpha,\beta$-difructofuranose dianhydrides. Pour des temps de réaction courts, de deux heures par exemple le rendement total, estimé par $^{13}$C-r.m.n., en dérivés dianhydrides (5) et (6) est de l'ordre de 75 % et la proportion relative des composants $\beta,\beta$ (5) et $\alpha,\beta$ (6) de l'ordre de 29 et 47 %, le reste du mélange réactionnel étant constitué d'un peu de dérivés acétylés des fluorures de fructofuranosyle et de glucopyranosyle (schéma de la figure 1). Lorsque le temps de réaction augmente, la proportion de l'isomère $\alpha,\beta$-difructofuranose dianhydride (6) augmente corrélativement, au regard de l'isomère $\beta,\beta$ (5). C'est ainsi qu'un temps de réaction de 17 h permet d'obtenir un rendement de 71 % en hexa-O-acetyl $\alpha$-$\underline{D}$-fructofuranose $\beta$-$\underline{D}$-fructofuranose, 1,2':2,1'-dianhydride (6) (62 % en produit isolé), à côté de seulement 11 % de son isomère $\beta,\beta$(5). Dans le cas où l'obtention du dianhydride cristallin $\alpha,\beta$ (6) est souhaitée, c'est évidemment cette technique qu'on préfère. Au-delà de ce temps de réaction, le rendement en dianhydrides (5) et (6) formés diminue du fait de la formation de produits autres qui n'ont pas été davantage identifiés.

Le principe de cette réaction étant basé, selon le schéma de la figure 1, sur la dimérisation du fructose stabilisé par des substituants acétate sous sa forme furanose, par réaction nucléophile de l'hydroxyle primaire en C-1 d'une première molécule sur le centre anomère d'une seconde molécule, il va de soi que le traitement sous les mêmes conditions de tout dérivé de l'inuline impliquant une protection des hydroxyles en C-3, C-4 et C-6 par un groupement stable dans le fluorure d'hydrogène, comme des substituants ester par exemple, conduira à la formation de dianhydrides du fructofuranose. De même, tout composé permettant l'obtention, par hydrolyse acide, d'un cation fructofuranosyle protégé sur les positions C-3, C-4 et C-6 par un groupement protecteur stable dans le fluorure d'hydrogène, sera également adapté à la production de dianhydrides du fructofuranose et entre dans le cadre de cette invention. C'est le cas en particulier d'acétals 2,1':4,6 du saccharose, préparés entre autre par acétonation cinétique du saccharose par utilisation de 2-alcoxy-propènes selon E. Fanton, J. Gelas, D. Horton, H. Karl, R. Khan, C. Kuan Lee et G. Patel (J. Org. Chem., 46 (1981) 4057-4060) ou de dérivés du diméthoxydiphénylsilane impliquant ces mêmes positions (M.R. Jenner et R. Khan, J Chem. Soc., Chem. Commun., 1980, 50-51), puisqu'il est possible de substituer les groupes hydroxyle restants par des groupes acyle stables dans le fluorure d'hydrogène. Ces concepts s'appliquent également aux glucides correspondants de la série $\underline{L}$.

Ces composés (7) et (8) sont notamment caractérisés par leurs spectres de $^{13}$C-r.m.n. qui montrent les signaux respectifs suivants :

7 : $\delta$ (p.p.m.), 104.55 (C-2), 83.68, 80.76, 77.56 (C-3,C-4,C-5), 61.82 (C-1,C-6).

8 : $\delta$ (p.p.m.), 103.3, 99.7 (C-2,C-2'), 84.3, 82.7, 82.1, 78.6, 77.8, 75.4 (C-3,C-4,C-5,C-3',C-4',C-5'), 63.5, 63.4, 62.6, 62.0 (C-1,C-6,C-1',C-6'),

par leur temps de rétention en chromatographie liquide sous pression (CLHP, colonne Sugar Pak (Waters, éluant eau, 93°C) ; tr 7, 3.9 ; tr 8, 4.0, ainsi que par leurs constantes physico-chimiques : 7, non cristallin, $(\alpha)_D^{25}$ + 92,7° (C 2.18, eau) ; 8, p.f. 161-162°,$(\alpha)_D^{22}$ +27 (c 1, eau).

Les $\underline{D}$-glucose et $\underline{D}$-fructose, éventuellement résiduels, sont dosés par les méthodes enzymatiques commercialisées par la Société Boehringer Mannheim.

Les exemples suivants illustrent l'invention :

EXEMPLE 1 :

Dans un récipient en polyéthylène, le peracétate d'inuline (100 g) est additionné, à -25° et agitation, d'un mélange de fluorure d'hydrogène (100 mL) et de dioxyde de soufre (100 mL) et la solution obtenue est maintenue à cette température pendant 2 h. Elle est alors refroidie dans l'azote liquide et additionnée de dichlorométhane (500 mL), de glace et d'eau (300 mL) et neutralisée par addition d'hydrogénocarbonate de sodium (470 g). La phase dichlorométhylénique est séparée par décantation et la phase aqueuse est à nouveau lavée par le dichlorométhane. Les extraits dichlorométhyléniques réunis sont séchés sur sulfate de sodium et concentrés sous pression réduite sous forme d'un résidu huileux (100 g). L'étude réalisée en $^{13}$C-r.m.n. sur ce produit brut monte qu'il est composé essentiellement de fluorure de $\underline{D}$-fructofuranosyle partiellement acétylé (24 %), d'hexa-O-acetyl $\alpha$-$\underline{D}$-fructofuranose-$\beta$- $\underline{D}$-fructofuranose 1,2':2,1'-dianhydride (47 %), et d'hexa-O-acétyl di-$\beta$-$\underline{D}$-fructofuranose 1,2':2,1'-dianhydride (29 %).

Ce résidu huileux est dissous dans l'éther et le composant hexa-O-acetyl $\alpha$-$\underline{D}$-fructofuranose $\beta$-$\underline{D}$-fructofuranose 1,2':2,1'- dianhydride, qui cristallise spontanément, est séparé par filtration (25 g, 25 %) - p.f. 124-125° et quelquefois 135-136° (possible dimorphisme) ; $(\alpha)_D^{20}$ -0.9 (c 5.5, chloroforme).

L'évaporation des eaux-mères permet d'obtenir l'autre isomère, l'hexa-O-acétyl-di-$\beta$-$\underline{D}$-fructofuranose 1,2':2,1'-dianhydride, obtenu sous la forme d'une substance huileuse en mélange avec du fluorure de $\underline{D}$-fructofuranosyle partiellement acétylé.

La dé-O-acétylation des dérivés hexa-O-acétates précédents est réalisée par dissolution dans le méthanol sec (100 mL) et addition d'une solution méthanolique de méthylate de sodium (M, 20 mL). Après 18 h à température ambiante, la solution est déminéralisée par passage sur une colonne échangeuse mixte cations, anions (MB-3). L'évaporation conduit, pour l'$\alpha$-$\underline{D}$-fructofuranose $\beta$-$\underline{D}$-fructofuranose 1,2':2,1'-dianhydride,à un solide qui cristalline par redissolution dans l'éthanol (14 g, 100 %) ; p.f. 161-162° ; $(\alpha)^{22}$ +27° (c 1, eau). L'autre isomère di-$\beta$-$\underline{D}$-fructofuranose-1,2':2,1' dianhydride est obtenu sous la forme d'une substance huileuse, en mélange avec du méthyl $\underline{D}$-fructofuranoside.

Note : Le peracétate d'inuline peut être préparé selon Haworth et Streight (Helv. Chim. Acta, 15 (1932) 609-615), ou de préférence selon la technique suivante :

L'inuline (100 g) est additionné à un mélange d'anhydride acétique (100 mL) et de pyridine (100 mL) et la suspension est portée à ébullition pendant 24 h. Elle est ensuite concentrée sous pression réduite et le résidu visqueux et noir est co-évaporé en présence de méthanol (100 mL), de façon à éliminer la pyridine et l'anhydride acétique résiduels. L'addition de méthanol conduit au peracétate d'inuline cristallin identique à la littérature (loc. cit.)

EXEMPLE 2 :

Le mode opératoire précédent est suivi, sauf pour ce qui concerne la température de la réaction du mélange HF-SO$_2$ qui est de -20° et le temps de cette réaction est porté à 3 h. Un spectre de $^{13}$C-r.m.n. du mélange total après extraction indique la présence des composants suivants :
- Fluorure de $\underline{D}$-fructofuranosyle partiellement acétylé (20 %).
- Hexa-O-acétyl $\alpha$-$\underline{D}$-fructofuranose $\beta$-$\underline{D}$-fructofuranose 1,2':2,1'-dianhydride (60 %).
- Hexa-O-acétyl di-$\beta$-$\underline{D}$-fructofuranose 1,2':2,1'-dianhydride (19%).

Partant de 120 g d'acétate d'inuline, on obtient 51,8 g (43 %) d'hexa-O-acétyl $\alpha$-$\underline{D}$-fructofuranose $\beta$-$\underline{D}$-fructofuranose 1,2':2,1'-dianhyride cristallisé, qui conduit par désacétylation à 29 g (100 %) d'$\alpha$-$\underline{D}$-fructofuranose $\beta$-$\underline{D}$-fructofuranose 1,2':2,1'-dianhydride cristallisé.

EXEMPLE 3:

Le mode opératoire de l'exemple 1 est suivi, mais le temps de contact du mélange HF-SO$_2$-triacétate d'inuline est porté à 17 h (à -20°). Un spectre de $^{13}$C-r.m.n. du mélange total montre la présence des composants suivants :
- Fluorure de $\underline{D}$-fructofuranosyle partiellement acétylé (18 %).
- Hexa-O-acétyl $\alpha$-$\underline{D}$-fructofuranose $\beta$-$\underline{D}$-fructofuranose 1,2':2,1'-dianhydride (71 %).
- Hexa-O-acétyl di-$\beta$-$\underline{D}$-fructofuranose 1,2':2,1'-dianhydride (11 %).

Partant de 100 g d'acétate d'inuline, on obtient 62 g (62 %) d'hexa-O-acétyl $\alpha$-$\underline{D}$-fructofuranose $\beta$-$\underline{D}$-fructofuranose dianhydride cristallisé qui conduit par désacétylation à 35 g (100 %) d'$\alpha$-$\underline{D}$- fructofuranose $\beta$-$\underline{D}$-fructofuranose 1,2':2,1'-dianhydride cristallisé.

EXEMPLE 4 :

Le mode opératoire de l'exemple 1 est suivi, mais le temps de contact du mélange HF-SO$_2$-triacétate d'inuline est porté à 24 h. Un spectre de $^{13}$C-r.m.n. du mélange total montre la présence des composants suivants :
- Fluorure de $\underline{D}$-fructofuranosyle partiellement acétylé (10 %).
- Hexa-O-acétyl $\alpha$-$\underline{D}$-fructofuranose $\beta$-$\underline{D}$-fructofuranose 1,2':2,1'-dianhydride (47 %).
- Hexa-O-acétyl di-$\beta$-$\underline{D}$-fructofuranose 1,2':2,1'-dianhydride (43 %).

Partant de 100 g d'acétate d'inuline, on obtient 35 g (35 %) d'hexa-O-acétyl $\alpha$-$\underline{D}$-fructofuranose $\beta$-$\underline{D}$-fructofuranose dianhydride cristallisé et 19,5 g (100 %) d'$\alpha$-$\underline{D}$-fructofuranose $\beta$-$\underline{D}$-fructofuranose 1,2':2,1'-dianhydride cristallisé.

EXEMPLE 5 :

Le mode opératoire de l'exemple 1 est suivi mais le dioxanne remplace le dioxyde de soufre. Pour 100 g de peracétate d'inuline, on utilise 400 mL de fluorure d'hydrogène et 100 mL de dioxanne et le mélange réactionnel est maintenu pendant 1h30 à la température de 20-25°. L'extraction conduit à une huile qui cristallise dans l'éther

Partant de 100 g d'acétate d'inuline, on obtient 20 g (20 %) d'hexa-O-acétyl α-D̲-fructofuranose β-D̲-fructofuranose dianhydride cristallisé qui conduit par désacétylation à 11,2 g (100 %) d'α-D̲- fructofuranose β-D̲-fructofuranose 1,2′:2,1′-dianhydride cristallisé.

EXEMPLE 6 :

Les modes opératoires décrits dans les exemples 1 à 4 sont repris jusqu'à l'étape d'extraction des mélanges réactionnels par le dichlorométhane, étape suivie d'évaporation des extraits dichlorométhyléniques sous forme de résidus huileux. Ces derniers sont alors dissous dans l'éthanol (1500 mL), avec l'aide si nécessaire d'un léger chauffage (40°), et additionnés d'une solution d'hydroxyde de sodium dans l'eau (400 mL, 6 % p/v). Les solutions sont maintenues à 40° pendant 0.5 h, puis déminéralisées par passage sur une colonne de résine échangeuse mixte de cations-anions (MB-3). Les solutions, ainsi déminéralisées, sont d'abord concentrées sous pression réduite, puis lyophilisées. Des substances vitreuses incolores sont obtenues ( 53 g, 95 % partant de peracétate d'inuline, 100 g). Une analyse en $^{13}$C-r.m.n. ne permet pas de détecter, dans aucun de ces échantillons, la présence d'éthylglycosides. Une analyse des oses libres résiduels donne les résultats suivants pour chacun des échantillons :

| Temps de réaction | Oses résiduels | |
|---|---|---|
| | D̲-glucose | D̲-fructose |
| 2 h (Exemple 1) | entre 0,8 et 1,5 % | 9% |
| 3 h (Exemple 2) | " | 9% |
| 17 h (Exemple 3) | " | 10% |
| 24 h (Exemple 4) | " | 13% |

**Revendications**

1.  Procédé de préparation de dianhydrides dispiro-dioxanniques dérivés du fructofuranose caractérisés en ce que l'on fait réagir un glucide comportant au minimum un résidu fructofuranose, substitué sur les positions hydroxyles en C-3, C-4 et C-6 par des fonctions stables en présence de fluorure d'hydrogène, avec le fluorure d'hydrogène utilisé comme solvant du glucide, et réactif. Cette étape est suivie d'une déprotection des positions hydroxyles protégées.

2.  Procédé, selon la revendication 1, caractérisé en ce qu'un cosolvant tel que le dioxyde de soufre, le dioxanne ou tout autre diluant, faiblement ou non réactif vis à vis du fluorure d'hydrogène est incorporé au mélange réactionnel.

3.  Procédé, selon l'une des revendications 1 à 2, caractérisé en ce que la température de la réaction est comprise entre -20° et 0° ou, dans le cas du dioxanne, la température ambiante.

4.  Procédé, selon l'une des revendications 1 à 3, caractérisé en ce que le glucide est l'inuline.

5.  Procédé, selon l'une des revendications 1 à 3, caractérisé en ce que le glucide est le saccharose.

6.  Procédé, selon l'une des revendications 1 à 5, caractérisé en ce que les groupes protecteurs stables

EP 0 252 837 B1

en présence de fluorure d'hydrogène, sont des groupes esters.

7. Procédé, selon la revendications 6, caractérisé en ce que le glucide substitué est le triacétate d'inuline.

8. Procédé, selon la revendication 7, caractérisé en ce que l'on contrôle le temps de réaction en fonction de la proportion relative des composants du mélange réactionnel que l'on désire obtenir.

9. Procédé, selon l'une des revendications 7 et 8, caractérisé en ce que l'on sépare par cristallisation le 3, 4, 6:3′,4′,6′-hexa-O-acetyl α-D̲- fructofuranose β-D̲-fructofuranose 1,2′:2,1′:dianhydride du mélange réactionnel résultant et qu'on le désacétyle par action d'une base de manière à obtenir le composé déprotégé correspondant α-D̲-fructofuranose β- D̲-fructofuranose 1,2′:2,1′-dianhydride.

10. Procédé, selon l'une des revendications 7 et 8, caractérisé en ce que l'on désacétyle directement le produit de la réaction de manière à obtenir les dianhydrides α-D̲- fructofuranose β-D̲-fructofuranose 1,2′:2,1′-dianhydride, et di-β-D̲-fructofuranose 1,2′:2,1′-dianhydride en mélange.

11. Di-β,β-D̲-fructofuranose dianhydride suceptible d'être obtenu selon l'une des revendications 1 à 10.

12. 3,4,6:3′,4′,6′-hexa-O-acetyl di-β -D̲-fructofuranose 1,2′:2,1′-dianhydride susceptible d'être obtenu selon l'une des revendications 1 à 10.

13. Applications des produits obtenus selon l'une des revendications 9 et 10 comme agents édulcorants à faible valeur calorique et non ou faiblement caryogènes.

**Claims**

1. A process for preparation of dispirodioxanic dianhydrides derived from fructofuranose, characterized in that a carbohydrate having a minimum of one fructofuranose residue, substituted on the C-3, C-4 and C-6 hydroxyl positions by groups stable in presence of hydrogen fluoride, is reacted with hydrogen fluoride used as solvent for the carbohydrate and as reactant. This step is followed by a deprotection of the protected hydroxyl positions.

2. A process according to Claim 1, characterized in that a co-solvent such as sulphur dioxide, dioxane or any other diluent with little or no reactivity towards hydrogen fluoride is incorporated in the reaction mixture.

3. A process according to one of Claims 1 to 2, characterized in that the reaction temperature is between -20° and 0° or, in the case of dioxane, ambient temperature.

4. A process according to one of Claims 1 to 3, characterized in that the carbohydrate is inulin.

5. A process according to one of Claims 1 to 3, characterized in that the carbohydrate is sucrose.

6. A process according to one of Claims 1 to 5, characterized in that the protecting groups stable in presence of hydrogen fluoride are ester groups.

7. A process according to Claim 6, characterized in that the substituted carbohydrate is inulin triacetate.

8. A process according to Claim 7, characterized in that the reaction temperature is controlled as a function of the relative proportion of the components of the reaction mixture which it is desired to obtain.

9. A process according to one of Claims 7 and 8, characterized in that 3,4,6:3',4',6'-hexa-O-acetyl α-D-fructofuranose β-D-fructofuranose 1,2':2,1'-dianhydride is separated by crystallization from the resulting reaction mixture and that it is deacetylated by the action of a base so as to obtain the corresponding deprotected compound, α-D-fructofuranose β-D-fructofuranose 1,2':2,1'-dianhydride.

10. A process according to one of Claims 7 and 8, characterized in that the reaction product is

6

deacetylated directly so as to obtain the dianhydrides α-D-fructofuranose β-D-fructofuranose 1,2':2,1'-dianhydride and di-β-D-fructofuranose 1,2':2,1'-dianhydride in a mixture.

11. Di-β-D-fructofuranose dianhydride as obtained according to one of Claims 1 to 10.

12. 3,4,6:3',4',6'-hexa-*O*-acetyl di-β-D-fructofuranose 1,2':2,1'-dianhydride as obtained according to one of Claims 1 to 10.

13. Applications of the products obtained according to one of Claims 9 and 10 as sweetening agents of low calorific value and of zero or low cariogenicity.

**Patentansprüche**

1. Verfahren zur Herstellung von dispiro-dioxanischen Dianhydrid-Derivaten der Fructofuranose, dadurch gekennzeichnet, daß man ein Kohlehydrat, das mindestens einen Fructofuranose-Rest aufweist, der an den Hydroxylpositionen von C-3, C-4 und C-6 mit stabilen Funktionen substituiert ist, in Gegenwart von Fluorwasserstoff mit dem als Lösungsmittel des Kohlehydrates und Reagenz verwendeten Fluorwasserstoff reagieren läßt, wonach diesem Schritt eine Deprotektion der geschützten Hydroxylpositionen folgt.

2. Verfahren nach Anspruch 1, dadurch gekennzeichnet, daß man in das Reaktionsgemisch ein weiteres Lösungsmittel, beispielsweise Schwefeldioxid, Dioxan oder irgendein anderes Verdünnungsmittel, das mit Fluorwasserstoff nur schwach oder überhaupt nicht reagiert, in das Reaktionsgemisch einarbeitet.

3. Verfahren nach einem der Ansprüche 1 oder 2, dadurch gekennzeichnet, daß man die Reaktionstemperatur zwischen -20° und 0° oder beim Dioxan auf Raumtemperatur hält.

4. Verfahren nach einem der Ansprüche 1 bis 3, dadurch gekennzeichnet, daß man als Kohlehydrat Inulin verwendet.

5. Verfahren nach einem der Ansprüche 1 bis 3, dadurch gekennzeichnet, daß man als Kohlehydrat Saccharose verwendet.

6. Verfahren nach einem der Ansprüche 1 bis 5, dadurch gekennzeichnet, daß man als in Gegenwart des Fluorwasserstoffs stabile Schutzgruppen Ester-Gruppen verwendet.

7. Verfahren nach Anspruch 6, dadurch gekennzeichnet, daß man als substituiertes Kohlehydrat Inulintriacetat verwendet.

8. Verfahren nach Anspruch 7, dadurch gekennzeichnet, daß man die Reaktionszeit in Abhängigkeit vom Anteil der Bestandteile des Reaktionsgemisches, den man erreichen will, steuert.

9. Verfahren nach einem der Ansprüche 7 und 8, dadurch gekennzeichnet, daß man durch Kristallisation das 3,4,6:3',4',6'-Hexa-O-Acetyl α-D̲-Fructofuranhose β-D̲-Fructofuranose 1,2':2,1'-Dianhydrid vom entstehenden Reaktionsgemisch abtrennt und durch Einwirkung einer Base desacetyliert, um den α-D̲-Fructofuranose β-D̲-Fructofuranose 1,2':2,1'-Dianhydrid entsprechenden ungeschützten Bestandteil zu erhalten.

10. Verfahren nach einem der Ansprüche 7 und 8, dadurch gekennzeichnet, daß man das Reaktionsprodukt direkt desacetyliert, um die Dianhydride α-D̲-Fructofuranose β-D̲-Fructofuranose 1,2':2,1'-Dianhydrid unddi-β-D̲-Fructofuranose 1,2';2,1'-Dianhydrid im Gemisch zu erhalten.

11. Di-β, β-D̲-Fructofuranose Dianhydrid erhalten nach einem der Ansprüche 1 bis 10.

12. 3,4,6:3',4'.6'-Hexa-O-Acetyl di-β-D̲-Fructofuranose 1,2':2,1'-Dianhydrid erhalten nach einem der Ansprüche 1 bis 10.

13. Verwendung der nach einem der Ansprüche 9 und 10 erhaltenen Produkte als Süßstoffe mit schwachem Kaloriengehalt und schwacher oder keiner kariogener Wirkung.

Figure 1- Schéma de préparation des dianhydrides du fructofuranose 7 et 8

R= Ac ou acyle